**Europäisches Patentamt**

**European Patent Office**

**Office européen des brevets**

(19)

(11) Veröffentlichungsnummer : **0 456 670 B1**

(12) **EUROPÄISCHE PATENTSCHRIFT**

(45) Veröffentlichungstag der Patentschrift :
18.11.93 Patentblatt 93/46

(51) Int. Cl.⁵ : **A61K 31/20, A61K 31/23, A61K 47/24, A61K 9/107**

(21) Anmeldenummer : **90902180.0**

(22) Anmeldetag : **31.01.90**

(86) Internationale Anmeldenummer :
**PCT/EP90/00171**

(87) Internationale Veröffentlichungsnummer :
**WO 90/08544 09.08.90 Gazette 90/19**

(54) **OMEGA-3-FETTSÄUREHALTIGE FETTEMULSION ZUR INTRAPERITONEALEN APPLIKATION, IHRE HERSTELLUNG UND ANWENDUNG.**

(30) Priorität : **02.02.89 DE 3903056**

(43) Veröffentlichungstag der Anmeldung :
**21.11.91 Patentblatt 91/47**

(45) Bekanntmachung des Hinweises auf die Patenterteilung :
**18.11.93 Patentblatt 93/46**

(84) Benannte Vertragsstaaten :
**AT BE CH DE DK ES FR GB IT LI LU NL SE**

(56) Entgegenhaltungen :
**EP-A- 0 298 293**
**EP-A- 0 311 091**

(73) Patentinhaber : **B. Braun Melsungen AG**
**Postfach 120**
**D-34209 Melsungen (DE)**

(72) Erfinder : **GEORGIEFF, Michael**
**Hintere Pfaffenleite 6**
**D-8551 Kunreuth (DE)**
Erfinder : **NEHNE, Jörg**
**Weserring 7**
**D-3501 Guxhagen (DE)**
Erfinder : **BOLL, Michael**
**Leipziger Str. 6**
**D-3508 Melsungen (DE)**

(74) Vertreter : **von Kreisler, Alek, Dipl.-Chem. et al**
**Patentanwälte, Von Kreisler-Selting-Werner,**
**Postfach 10 22 41, Bahnhofsvorplatz 1**
**D-50462 Köln (DE)**

**Beschreibung**

Die vorliegende Erfindung betrifft die Verwendung einer Fettemulsion zur Herstellung eines Arzneimittels zur intraperitonealen Applikation.

Neben den bekannten Zugangswegen per os und per infusionem wird seit mehreren Jahren auch die Bauchhöhle zur Verabreichung von Flüssigkeit und Elektrolyten (August D.A., Sugarbaker P.H. Surgery 97 (1985) 237), Medikamenten (Jenkins J., et al. Surg. Gynecol. Obstet. 154 (1982) 858; Schade D.S., et al. J. Clin. Endocrinol. Metab. 52 (1981) 1165) sowie Nährstoffen (Gilsdorf R.B., et al. J. Am. Coll. Nutr. 4 (1985) 461; Stone M.M., et al. J. Pediatr. Surg. 21 (1986) 267; Klein M.D., et al. J. Pediatr. Surg. 20 (1985) 765) genutzt. Besondere Beachtung hat die Bauchhöhle im Rahmen der Peritonealdialyse gefunden, die eine weltweit anerkannte Methode zur Behandlung der chronischen Urämie darstellt. Hauptsächlich im Rahmen dieser Indikation wurden Verfahren zum geeigneten und teilweise dauerhaften Zugang zur Bauchhöhle entwickelt (Ponce S.P., et al. Perit. Dial. Bull. 2 (1982) 82), und die Physiologie der Bauchhöhle sowie Diffusions- und Absorptionsvorgänge an der Peritonealmembran wurden eingehend erforscht. Die dabei gewonnenen Erfahrungen zeigen, daß die intraperitoneale Zufuhr einen zur Verabreichung pharmakologisch wie nutritiv wirksamer Substanzen gut praktikablen Weg darstellt.

Den zitierten Beispielen ist gemeinsam, daß es bei ihnen um die Erzielung systemischer Wirkungen auf intraperitonealem Weg geht. Daneben werden intraperitoneale Verfahren aber auch zur Behandlung von Erkrankungen der Bauchhöhle selbst eingesetzt, z.B. die Spülung der Bauchhöhle mit Ringer-Glukoselösungen bei Peritonitis. Solche Spülungen erfolgen in der Hauptsache, um infektiöses Material wie Eiter, Darminhalt usw. zu entfernen. Zum Teil gelangen dabei antibiotisch oder antiseptisch wirkende Zusätze zur Anwendung, beispielsweise Polyvidon-Jod, verschiedene Antibiotika oder Taurolin. Ob diese Zusätze über den rein mechanischen Effekt der Spülung hinaus eine günstige Wirkung auf die Behandlungsergebnisse haben, wird jedoch kontrovers beurteilt.

Wegen der besonderen Gefährlichkeit septischer Erkrankungen der Bauchhöhle, die je nach Ursache mit einer hohen Letalität von bis über 70 % verbunden sein können, besteht dringlicher Bedarf an neuen, prophylaktisch und therapeutisch wirksamen Konzepten.

Das Peritoneum mit seiner riesigen Oberfläche stellt ein ausgedehntes Immunsystem dar, das nach Trauma, Operation, Schock, Sepsis usw. nicht ausreichend perfundiert wird und infolgedessen die Ausweitung infektiöser Prozesse begünstigt. Ziel prophylaktischer und/oder therapeutischer Maßnahmen bei den genannten Erkrankungen muß es daher sein, die Immunabwehr und die allgemeine Perfusion des Peritoneums zu steigern, um damit die Voraussetzungen für eine erfolgreiche chirurgische Intervention und für das Ansprechen auf eine Antibiotikatherapie zu ermöglichen.

Der vorliegenden Erfindung liegt somit die Aufgabe zugrunde, die Bauchhöhle in geeigneter Weise mit nutritiv und pharmakologisch wirksamen Substraten zu versorgen.

Es wurde überraschenderweise gefunden, daß sich die oben genannten Aufgaben durch intraperitoneale Verabreichung einer Fettemulsion auf Basis von ω-3-Fettsäuren oder deren Ester lösen und die Mortalität septischer Erkrankungen der Bauchhöhle auf diese Weise deutlich vermindern lassen.

Die vorliegende Erfindung betrifft die Verwendung einer Fettemulsion, enthaltend ω-3-Fettsäuren, insbesondere Eikosapentaensäure (EPA), bwz. ihre physiologisch unbedenklichen Ester als Bestandteile der Fettphase, wobei die Fettemulsion

- ω-3-Fettsäuren, insbesondere EPA, bzw. ihre physiologisch unbedenklichen Ester in Reinform oder als Bestandteil von Fischölen oder Fischölfraktionen,
- mindestens einen physiologisch unbedenklichen Emulgator,
- gegebenenfalls weitere Fette wie mittelkettige Triglyzeride (MCT),
- gegebenenfalls α-Tocopherol oder physiologisch unbedenkliche α-Tocopherolester,
- gegebenenfalls Ascorbinsäure oder physiologisch unbedenkliche Ascorbinsäureester sowie
- übliche Zusatz- und Hilfsstoffe enthält, wobei
- der gesamte Fettgehalt zwischen 5 und 20 % liegt und
- der Emulgatorgehalt zwischen 5 und 12 % (bezogen auf den Fettgehalt) liegt,

zur Herstellung eines Arzneimittels zur intraperitonealen prophylaktischen und therapeutischen Behandlung septischer Erkrankungen der Bauchhöhle.

ω-3-Fettsäuren und deren Ester, insbesondere aber Eikosapentaensäure (EPA), bzw. ihre physiologisch unbedenklichen Ester können erfindungsgemäß entweder in Reinform oder als Bestandteil von Fischölen bzw. Fischölfraktionen verwendet werden.

Physiologisch und pharmakologisch unbedenklich sind die niederen Alkylester bzw. Glyzerinester von ω-3-Fettsäuren, insbesondere EPA. Bevorzugt werden die Ethylester und Triacylglyzerole.

Geeignete Fischöle sind beispielsweise solche, wie sie technisch in bedeutendem Umfang aus Kaltwas-

EP 0 456 670 B1

serfischen gewonnen werden. Bevorzugt sind hochgereinigte Fischölkonzentrate oder Fischölfraktionen, die beispielsweise aus Makrele, Sardine, Hering oder Lachs gewonnen werden, wobei diese einen EPA-Gehalt von vorzugsweise mindestens 25 % (bezogen auf die Fettsäuremethylester des Fischölkonzentrats) besitzen.

Als Eventualkomponente "weitere Fette" werden erfindungsgemäß mittelkettige Triglyzeride verwendet, die zu mindestens 90 % aus Glyzeriden der Caprylsäure und Caprinsäure bestehen. Der Gehalt dieser Komponenten liegt (bezogen auf den lipophilen Anteil der Emulsion) bei 0 bis 90 %.

Als Emulgatoren werden physiologisch unbedenkliche Emulgatoren wie Phospholipide tierischen und pflanzlichen Ursprungs verwendet, insbesondere solche aus Hühnereigelb oder Soja.

Der Emulgatorgehalt beträgt 5 bis 12 % (bezogen auf den Fettgehalt) und der gesamte Fettgehalt der Fettemulsion beträgt zwischen 5 und 20 %.

Als Emulgierhilfsstoffe können weiterhin Natriumsalze langkettiger Fettsäuren (bevorzugt in einer Konzentration von 0,005 bis 0,1 Gew.-%, bezogen auf die Gesamtemulsion) und/oder Cholesterin oder Cholesterinester (bevorzugt 0,005 bis 0,1 Gew.-%, bezogen auf die Gesamtemulsion) verwendet werden.

Die Fettemulsion weist vorzugsweise einen pH-Wert im Bereich von 6 bis 9 auf.

Weiterhin kann der Fettemulsion gegebenenfalls $\alpha$-Tocopherol oder $\alpha$-Tocopherol-Ester in einer Menge von 0 bis 100 mg, bezogen auf 100 g Fett, zugegeben werden, insbesondere wenn $\omega$-3-Fettsäuren, insbesondere EPA, bzw. ihre Ester in Reinform verwendet werden.

Schließlich können der Fettemulsion gegebenenfalls außerdem Ascorbinsäure oder physiologisch unbedenkliche Ascorbinsäureester in einer Menge von 0 bis 500 mg, bezogen auf 100 g Fett, zugegeben werden.

Die Herstellung einer Fettemulsion zur intraperitonealen Applikation geschieht in der Weise, daß man die vorstehend beschriebenen Komponenten zunächst dispergiert und daraufhin homogenisiert.

Die vorliegende Erfindung findet vorzugsweise Anwendung nach Traumen und nach Operationen im Abdominalbereich, bei Sepsis, Peritonitis und hämorrhagisch nekrotisierender Pankreatitis, bei intraabdominellen Spülbehandlungen sowie Spüldrainagen. Weiterhin verwendet man die Fettemulsion zur intraperitonealen Ernährung. Man setzt diese Erfindung insbesondere bei vorstehend genannten Indikationen zur Prophylaxe eines eventuellen Nierenversagens sowie therapeutisch zur Verbesserung der Splanchnikusperfusion ein.

In den im folgenden näher beschriebenen Untersuchungen zeigte sich, daß Ratten, die vor bzw. nach einer experimentell herbeigeführten Peritonitis mit Eikosapentaensäureethylester auf intraperitonealem Weg behandelt worden waren, eine signifikant höhere Überlebensrate aufwiesen als Kontrolltiere in zwei Gruppen, von denen die eine konventionelle Fettsäureester (Intra-lipid$^R$ = Ester von gesättigten Fettsäuren und $\omega$-6-Fettsäuren), die andere Kochsalzlösung (als Plazebo) erhalten hatte. In der Eikosapentaensäureethylester-Gruppe wurden signifikant niedrigere Konzentrationen an $TXB_2$, einem stabilen Metaboliten des Thromboxans $TXA_2$, beobachtet. $TXA_2$ ist ein außerordentlich potenter Mediator, der vor allem im Postaggressions- und septischen Stoffwechsel durch seine stark proaggregatorischen und vasokonstriktorischen Eigenschaften unerwünschte Wirkungen entfaltet. So trägt er entscheidend zu der ungenügenden Perfusion des Splanchnikusgebietes bei Sepsis bei. Die verminderte Konzentration von $TXB_2$ in der Eikosapentaensäureethylester-Gruppe kann als Beweis für eine Hemmung der Freisetzung und des oxidativen Stoffwechsels von Arachidonsäure, der Präkursorfettsäure für $TXA_2$, durch die Zufuhr kompetitiv wirkender $\omega$-3-Fettsäuren wie der Eikosapentaensäure aufgefaßt werden. Auf dem gleichen Wege können durch $\omega$-3-Fettsäuren auch stark inflammatorische ($LTB_4$) und immunsuppressive ($PGE_2$) Eikosanoide antagonisiert werden, die ebenfalls aus der Arachidonsäure entstehen, und deren Nachweise im Rahmen der beschriebenen Untersuchungen aus technischen Gründen nicht möglich war. So wie die reduzierte Bildung von $TXA_2$ zu einer verbesserten Splanchnikusperfusion führt, kann die Hemmung von $PGE_2$ und $LTB_4$ eine Steigerung der Immunkompetenz bewirken und im Sinne der gestellten Aufgabe zu einer Prophylaxe bzw. Therapie der Peritonitis und ihrer deletären Auswirkungen auf die Funktion vital wichtiger Organsysteme beitragen. Die neben den biochemischen Veränderungen beobachtete deutliche Verbesserung von Überlebenszeit und Überlebensrate in der Eikosapentaensäureethylester-Gruppe belegt, daß die intraperitoneale Verabreichung von $\omega$-3-Fettsäuren in dieser Weise wirksam ist.

Die Beobachtung, daß unerwünschte Wirkungen von Eikosanoiden, die sich aus $\omega$-6-Fettsäuren ableiten, durch $\omega$-3-Fettsäuren und den aus ihnen entstehenden Eikosanoiden antagonisiert werden können, ist nicht neu und hat bereits zu einer Reihe von Patentanmeldungen geführt.

Das US-Patent 4 526 902 beschreibt Mischungen aus 25 bis 75 Gew.-% Eikosapentaensäure und/oder Docosahexaensäure und einer $\omega$-6-Fettsäure, die enteral als Bestandteil von Pharmazeutika oder fetthaltigen Nahrungsmitteln wie Butter oder ähnlichem verwendet werden.

In der EP 0 120 169 Bl sind synthetische Triglyzeride beschrieben, die am mittleren C des Glyzerinmoleküls eine mehrfach ungesättigte Fettsäure, vorzugsweise Eikosapentaensäure oder Docosahexaensäure, besitzen können. Die so erzeugten Glyzeride können als Nahrungsmittel, Nahrungsergänzungsmittel oder als Arzneimittel für die therapeutische Ernährung verwendet werden.

Die JP-OS Sho-58-230918 beschreibt eine Eikosapentaensäure enthaltende Emulsion zur oralen und

3

nicht-oralen Verwendung. Diese enthält 1 bis 40 w/v-% Eikosapentaensäure und/oder Docosahexaensäure bzw. vorzugsweise deren Methyl- oder Ethylester, 1 bis 30 w/v-% eines Pflanzenöls, vorzugsweise Sojaöl, 0,01 bis 30 w/v-% α-Tocopherol, und als Emulgatoren 0,1 bis 5 w/v-% eines Phospholipids, vorzugsweise aus Eigelb und/oder Soja, sowie 0,1 bis 10 w/v-% eines nichtionischen synthetischen Emulgators.

Die DE-OS 34 09 793 offenbart eine flüssige Emulsion zur Transfusion mit antithrombotischer und antiarteriosklerotischer Wirkung, die zur Nahrungsergänzung dienen kann. Sie besteht neben Wasser zu 5 bis 20 w/v-% aus Eikosapentaensäure, Docosahexaensäure oder deren Ester und sie ist vorzugsweise ein gereinigtes Fischöl, wie Sardinenöl. Weiterhin enthält sie 1 bis 19 w/v-% eines Pflanzenöls, vorzugsweise Soja- und/oder Safloröl, sowie 1 bis 2 w/v-% eines Phospholipidemulgators, vorzugsweise aus Eigelb oder Soja. Dieser Fettemulsion kann als Antioxidans α-Tocopherol zugefügt werden.

WO 87/02247 beschreibt Fettemulsionen, enthaltend Fischöle mit hohem Anteil an ω-3-Fettsäureestern, die zur intravenösen Anwendung bei der Behandlung thrombotischer Erkrankungen vorgesehen sind.

In der EP 0 271 909 wird die Herstellung synthetischer Triglyzeride beschrieben, die als Fettsäuren solche mit einer Kettenlänge von 6 bis 12 C-Atomen, 14 bis 18 C-Atomen (vorzugsweise Linolsäure) sowie 20 bis 22 C-Atomen (vorzugsweise Eikosapentaensäure) in variablen Kombinationen und Relationen enthalten.

SE 8705122-A0 beschreibt ein Verfahren zur Herstellung einer intravenösen Fettemulsion, die ω-3-Fettsäuren als Bestandteil des Emulgators enthält.

Die DE-OS 37 34 147 betrifft eine isotone Fettemulsion zur parenteralen Applikation, enthaltend ω-3-Fettsäuren, insbesondere Eikosapentaensäure (EPA) oder ihre physiologisch unbedenklichen Ester als Bestandteile der Fettphase, die dadurch gekennzeichnet ist, daß die Fettemulsion

- diese ω-3-Fettsäuren oder Ester in Reinform oder als Bestandteil von Fischölen,
- mittelkettige Triglyzeride (MCT),
- mindestens einen physiologisch unbedenklichen Emulgator,
- gegebenenfalls mindestens ein ω-6-Fettsäuren lieferndes Pflanzenöl,
- α-Tocopherol oder physiologisch unbedenkliche α-Tocopherolester, sowie
- übliche Zusatz- und Hilfsstoffe

enthält, wobei

- das Verhältnis von EPA oder ihren physiologisch unbedenklichen Estern zu MCT zwischen 1 : 9 und 3 : 5 liegt,
- der gesamte Fettgehalt zwischen 5 und 30 % liegt,
- der Emulgatorgehalt zwischen 5 und 12 % (bezogen auf den Fettgehalt) liegt und
- der Gehalt an ω-6-Fettsäuren liefernden Pflanzenölen zwischen 0 und 30 % (bezogen auf den Fettgehalt) liegt.

In diesen Schriften wird die systemische, orale oder parenterale Anwendung von ω-3-Fettsäuren vorgeschlagen, um antithrombotische und antiarteriosklerotische Wirkungen zu erzielen bzw. um über eine Modulation des Eikosanoidwirkspektrums das Stoffwechselgeschehen bei Trauma oder Sepsis zu beeinflussen.

Neu an der vorliegenden Erfindung ist die in dieser Weise nicht vorhersehbare Beobachtung, daß ω-3-Fettsäuren auch intraperitoneal angewendet werden können, und daß sie so binnen kurzer Zeit eine besonders intensive prophylaktische und therapeutische Wirkung bei der Behandlung septischer Erkrankungen der Bauchhöhle entfalten.

Die Modulation der Eikosanoidwirkungen durch intraperitoneal verabreichte ω-3-Fettsäuren kann gegebenenfalls auch als Adjuvansbehandlung zur Antibiotikaprophylaxe oder Antibiotikatherapie angesehen werden. Mit der bisher geübten chirurgischen Praxis der Peritoneallavage ist die intraperitoneale Anwendung von ω-3-Fettsäuren hervorragend zu kombinieren; sie stellt so eine pharmakologische Ergänzung zu dem rein mechanischen Effekt der Spülung dar. Im Vergleich mit der systemischen Zufuhr lassen sich bei intraperitonealer Anwendung von ω-3-Fettsäuren die wirksame Dosis vermindern und weniger erwünschte systemische Effekte vermeiden. Indem sie unmittelbar an den Ort des Krankheitsgeschehens, den septischen Herd, gebracht werden, erlauben intraperitoneal verabreichte ω-3-Fettsäuren eine exaktere und enger am Therapieerfolg orientierte Dosierung als bei der Zufuhr auf anderen Wegen.

Die Wirkung der erfindungsgemäßen Verabreichung einer ω-3-fettsäurenhaltigen Fettemulsion zeigen die unten geschilderten Experimente. Weiterhin sind im folgenden Herstellungsbeispiele wiedergegeben.

Experiment 1:

36 Sprague-Dawley-Ratten wurden in Stoffwechselkäfigen bei einer Raumtemperatur von 22 °C, einer Luftfeuchtigkeit von 70 % sowie bei Fütterung mit Standardfutter gehalten und drei Tage auf die geplanten Untersuchungen vorbereitet. Nach der Eingewöhnungsphase wurden die Tiere in drei Gruppen zu jeweils 12 Ratten randomisiert und erhielten an drei aufeinanderfolgenden Tagen jeweils eine der nachstehenden Fettemulsionen (oder Kochsalzlösung in der Kontrollgruppe) als Instillation in die Bauchhöhle (in den linken unteren Quadranten des Abdomens):

Gruppe I:      (Plazebogruppe) physiologische Kochsalzlösung (30 ml/kg KG und Tag)

Gruppe II:     10 %ige Eikosapentaensäureethylester-Emulsion gemäß Herstellungsbeispiel 5 (30 ml/kg KG und Tag)

Gruppe III:    10 % Intralipid[R];
               Zusammensetzung: 100 g fraktioniertes Sojaöl, 12 g fraktioniertes Eilecithin und 22,5 g Glycerin, mit destilliertem Wasser auf 1000 ml ergänzt;
               (30 ml/kg KG und Tag).

24 h nach der letzten Injektion wurde bei den Tieren in Barbituratanästhesie das Abdomen eröffnet, das Zoekum ligiert, durch eine 18-Gauge-Nadel perforiert und auf diese Weise eine Peritonitis herbeigeführt (WICHTERMANN et al., J. Surg. Res. 29 (1980) 189-201). Bei der Perforation wurde auf Erhalt der Darmkontinuität geachtet. 16 h nach Zoekumligation und Zoekumperforation wurden die Konzentrationen ausgewählter Prostaglandinmetabolite im Serum der Tiere sowie im weiteren Verlauf die Überlebensraten bestimmt.

Ergebnisse:

|                                   | 24 h | 48 h | 72 h |
|-----------------------------------|------|------|------|
| Gruppe I   (Kochsalz)             | 9    | 6    | 4    |
| Gruppe II  (EPA-Ethylester)       | 12   | 10   | 8    |
| Gruppe III (Intralipid[R])        | 7    | 3    | 1    |

Tab. 1)    Zahl der Tiere (von jeweils n = 12 zu Versuchsbeginn), die zum angegebenen Zeitpunkt nach Zoekumligation und Zoekumperforation noch lebten.

|                                   | $TXB_2$ (ng/ml) |
|-----------------------------------|-----------------|
| Gruppe I   (Kochsalz)             | $15,7 \pm 3,8$  |
| Gruppe II  (EPA-Ethylester)       | $6,6 \pm 2,8*$  |
| Gruppe III (Intralipid[R])        | $23,2 \pm 4,1$  |

* $p < 0,01$ vs. Gr. I und Gr. III

Tab. 2)    Konzentration von $TXB_2$ im Serum 16 h nach Zoekumligation und Zoekumperforation.

Experiment 2:

In einem weiteren Experiment wurde überprüft, ob die erfindungsgemäße Fettemulsion auch dann noch wirkt, wenn sie erst nach Zoekumligation und Zoekumperforation verabreicht wird, also nach experimenteller Induzierung der Peritonitis. Für diese Untersuchung wurden ebenfalls Sprague-Dawley-Ratten herangezogen (n = 50), die nach Zoekumligation und Zoekumperforation wie in Experiment 1 näher beschrieben in 2 Gruppen zu je 25 Tieren randomisiert wurden. Tiere der Gruppe I erhielten insgesamt 15 mg/kg KG einer 20 %igen han-

5

delsüblichen Fettemulsion (Intralipid$^R$) und Tiere der Gruppe II erhielten 15 ml/kg KG einer 20 %igen Emulsion aus hochgereinigtem Fischöl gemäß dem Herstellungsbeispiel 4 als einmalige Instillation in die Bauchhöhle.

Ergebnisse:

|  | | 24 h | 48 h | 72 h |
|---|---|---|---|---|
| Gruppe I | (Intralipid$^R$) | 18 | 6 | 0 |
| Gruppe II | (Fischöl) | 22 | 16 | 11 |

Tab. 3)     Überlebensrate der Tiere 24, 48 und 72 h nach Induzierung der Sepsis.

Neben signifikant längerer Überlebenszeit der Tiere mit der Emulsion aus hochgereinigtem Fischöl wurden in dieser Gruppe auch signifikant geringere Konzentrationen an $TXB_2$ im Serum gemessen, ähnlich wie in Experiment 1.

Besonders hervorzuheben ist, daß die Urinproduktion der Tiere mit der Emulsion aus hochgereinigtem Fischöl nicht vermindert war, wie in der Gruppe mit Intralipid$^R$ (s. Tabelle 4). Dieser Befund zeigt, daß die erfindungsgemäße Behandlung mit $\omega$-3-Fettsäuren auch eine Prophylaxe des Nierenversagens nach Traumen bzw. infolge einer Sepsis darstellt.

|  | | 0-24 h | 24-48 h | 48-72 h |
|---|---|---|---|---|
| Gruppe I | (Intralipid$^R$) | 16±9 | 12±6 | |
| Gruppe II | (Fischöl) | 34±11[1] | 27±9[1] | 21±11 |

[1] $p < 0,001$ vs. Gr.I

Tab. 4)     Mittlere Urinproduktionsrate/Ratte in (ml/24 h).

Herstellungsbeispiele

Beispiel 1:

Eine Mischung aus 1000 g hochgereinigtem Fischöl, 1000 g MCT, 2 g Cholesterinazetat, 120 g gereinigten Eiphospholipiden und 2 g Natriumstearat wird mittels eines Ultra-Turrax fein dispergiert. Mit Aqua ad iniectabilia, das 250 g Glyzerol und 5 mmol Natriumhydroxid enthält, wird unter Rühren auf 10 l aufgefüllt. Diese grobe Voremulsion wird in einem Hochdruckhomogenisator bei einem Druck von 392 bar (400 kg/cm²) homogenisiert. Nach Abfüllung in Glasflaschen geeigneter Qualität wird nach allgemein bekanntem Verfahren hitzesterilisiert. Die Teilchen der sterilen und pyrogenfreien Emulsion sind kleiner als 1 μm.

Beispiel 2:

Eine Mischung aus 25 g Eikosapentaensäureethylester, 75 g MCT, 12 g gereinigten Eiphospholipiden, 50 mg α-Tocopherol und 0,2 g Natriumstearat wird, wie unter Beispiel 1 beschrieben, dispergiert und nach Auffüllen mit Aqua ad iniectabilia (enthält 25 g Glyzerol) auf 1000 ml homogenisiert, abgefüllt und sterilisiert. Die Emulsionstropfen sind kleiner als 1 μm.

Beispiel 3:

Eine Mischung aus 50 g Eikosapentaensäureethylester, 9 g gereinigten Eiphospholipiden, 50 mg α-Tocopherol, 100 mg Ascorbylpalmitat und 0,5 g Natriumstearat wird wie unter Beispiel 1 beschrieben dispergiert. Nach Auffüllen auf 1000 ml mit Aqua ad iniectabilia, das 25 g Glyzerol enthält, wird homogenisiert, abgefüllt und sterilisiert. Die Emulsionstropfen sind kleiner als 1 μm.

Beispiel 4:

Eine Mischung aus 2000 g hochgereinigtem Fischöl, 150 g gereinigten Eiphospholipiden, 500 mg α-Tocopherol, 2 g Ascorbylpalmitat sowie 3 g Natriumstearat wird wie unter Beispiel 1 beschrieben dispergiert und nach Auffüllen mit Aqua ad iniectabilia (enthält 250 g Glyzerol) auf 10 1 homogenisiert. Nach Einstellen des pH-Wertes mit Natriumhydroxid auf pH 8,0 bis 8,5 wird abgefüllt und sterilisiert. Die resultierenden Emulsionstropfen sind kleiner als 1 μm.

Beispiel 5:

Eine Mischung aus 100 g Eikosapentaensäureethylester, 12 g gereinigten Eiphospholipiden, 50 mg α-Tocopherol, 200 mg Ascorbylpalmitat und 0,5 g Natriumstearat wird dispergiert wie unter Beispiel 1 beschrieben. Anschließend wird mit Aqua ad iniectabilia, das 25 g Glyzerol enthält, auf 1000 ml aufgefüllt, homogenisiert, in Glasflaschen geeigneter Qualität abgefüllt und sterilisiert. Die Tropfen der so hergestellten Fettemulsion sind kleiner als 1 μm.

**Patentansprüche**

1. Verwendung einer Fettemulsion, enthaltend ω-3-Fettsäuren, insbesondere Eikosapentaensäure (EPA), bwz. ihre physiologisch unbedenklichen Ester als Bestandteile der Fettphase, wobei die Fettemulsion
   - ω-3-Fettsäuren, insbesondere EPA, bzw. ihre physiologisch unbedenklichen Ester in Reinform oder als Bestandteil von Fischölen oder Fischölfraktionen,
   - mindestens einen physiologisch unbedenklichen Emulgator,
   - gegebenenfalls weitere Fette wie mittelkettige Triglyzeride (MCT),
   - gegebenenfalls α-Tocopherol oder physiologisch unbedenkliche α-Tocopherolester,
   - gegebenenfalls Ascorbinsäure oder physiologisch unbedenkliche Ascorbinsäureester sowie
   - übliche Zusatz- und Hilfsstoffe enthält, wobei
   - der gesamte Fettgehalt zwischen 5 und 20 % liegt und
   - der Emulgatorgehalt zwischen 5 und 12 % (bezogen auf den Fettgehalt) liegt,
   zur Herstellung eines Arzneimittels zur intraperitonealen prophylaktischen und therapeutischen Behandlung septischer Erkrankungen der Bauchhöhle.

2. Verwendung der Fettemulsion gemäß Anspruch 1 zur Behandlung nach Traumen und nach Operationen im Abdominalbereich.

3. Verwendung der Fettemulsion gemäß Anspruch 1 zur Behandlung bei Sepsis, Peritonitis und hämorrhagisch nekrotisierender Pankreatitis.

4. Verwendung der Fettemulsion nach Anspruch 1 zur Behandlung im Rahmen intraabdomineller Spülbehandlungen sowie Spüldrainagen.

5. Verwendung der Fettemulsion gemäß Anspruch 1 zur Verbesserung der Splanchnikusperfusion nach operativen Eingriffen und Traumen.

6. Verwendung der Fettemulsion gemäß Anspruch 1 zur Prophylaxe des Nierenversagens nach operativen Eingriffen.

7. Verwendung der Fettemulsion gemäß Ansprüchen 1 bis 6, dadurch gekennzeichnet, daß das Fischöl ein hochgereinigtes Fischölkonzentrat mit einem Gehalt an EPA von vorzugsweise mindestens 25 % (bezogen auf die Fettsäuremethylester des Fischölkonzentrats) ist.

8. Verwendung der Fettemulsion gemäß Ansprüchen 1 bis 7, dadurch gekennzeichnet, daß dieses Fischöl/Fischölkonzentrat erhältlich ist durch Verarbeitung von Kaltwasserfischen, beispielsweise von Makrele, Sardine, Hering oder Lachs.

9. Verwendung der Fettemulsion gemäß Ansprüchen 1 bis 8, dadurch gekennzeichnet, daß die als Eventual-Fettkomponenten eingesetzten mittelkettigen Triglyceride zu mindestens 90 % aus Glyzeriden der Caprylsäure ($C_8$) und Caprinsäure ($C_{10}$) bestehen und der Gehalt dieser Komponente 0 bis 90 % (bezogen auf den lipophilen Anteil) beträgt.

10. Verwendung der Fettemulsion gemäß Ansprüchen 1 bis 9, dadurch gekennzeichnet, daß der eingesetzte Emulgator ein Phospholipid tierischen oder pflanzlichen Ursprungs, insbesondere aus Hühnereigelb oder Sojabohne, ist.

11. Verwendung der Fettemulsion gemäß Ansprüchen 1 bis 10, dadurch gekennzeichnet, daß sie weitere Emulgierhilfsstoffe wie Natriumsalze langkettiger Fettsäuren (bevorzugt 0,005 bis 0,1 Gew.-%, bezogen auf die Gesamtemulsion) und/oder Cholesterin oder Cholesterinester (bevorzugt 0,005 bis 0,1 Gew.-%, bezogen auf die Gesamtemulsion) enthält.

12. Verwendung der Fettemulsion gemäß Ansprüchen 1 bis 11, dadurch gekennzeichnet, daß sie 0 bis 100 mg $\alpha$-Tocopherol oder $\alpha$-Tocopherolester, bezogen auf 100 g Fett, enthält.

13. Verwendung der Fettemulsion gemäß Ansprüchen 1 bis 12, dadurch gekennzeichnet, daß sie 0 bis 500 mg Ascorbinsäure oder Ascorbinsäureester, bezogen auf 100 g Fett, enthält.

14. Verwendung der Fettemulsion gemäß Ansprüchen 1 bis 13, dadurch gekennzeichnet, daß sie einen pH-Wert im Bereich von 6 bis 9 aufweist.

## Claims

1. Use of a fat emulsion containing ω-3 fatty acids, and more specifically eicosapentaenoic acid (EPA) or its physiologically acceptable esters as components of the fat phase,
   wherein the fat emulsion contains
   - ω-3 fatty acids, and especially EPA, and/or its physiologically acceptable esters in the pure state or as component(s) of fish oils and/or fish oil fractions,
   - at least one physiologically acceptable emulsifier,
   - optionally further fats such as medium-chain tri-glycerides (MCT),
   - optionally α-tocopherol or physiologically acceptable α-tocopherol esters,
   - optionally ascorbic acid or physiologically acceptable ascorbic acid esters, as well as
   - conventional additives or auxiliary materials,
   whereby
   - the total fat content is between 5 and 20%, and
   - the emulsifier content is between 5 and 12% (based on the fat contents),
   for the preparation of a medicament for the intraperitoneal prophylactic and therapeutic treatment of septic diseases of the abdominal cavity.

2. Use of the fat emulsion according to claim 1 for the treatment after traumata and after surgery in the abdominal region.

3. Use of the fat emulsion according to claim 1 for the treatment of sepsis, peritonitis, and haemorrhagic necrotising pancreatitis.

4. Use of the fat emulsion according to claim 1 for the treatment in intra-abdominal lavage treatments and through drainages.

5. Use of the fat emulsion according to claim 1 for improving the splanchnic perfusion after surgery and traumata.

6. Use of the fat emulsion according to claim 1 for the prophylaxis of renal failure after surgery.

7. Use of the fat emulsion according to claims 1 to 6, characterized in that said fish oil is a highly purified fish oil concentrate having an EPA content of preferably at least 25% (based on the fatty acid methyl esters of the fish oil concentrate).

8. Use of the fat emulsion according to claims 1 to 7 characterized in that said fish oil/fish oil concentrate is obtainable by processing cold water fish such as, for example, mackerel, sardine, herring or salmon.

9. Use of the fat emulsion according to claims 1 to 8, characterized in that of the medium-chain triglycerides employed as optional components at least 90% consist of glycerides of caprylic ($C_8$) and capric ($C_{10}$) acids and the amount of this component is from 0 to 90% (relative to the lipophilic portion).

10. Use of the fat emulsion according to claims 1 to 9 characterized in that the emulsifier employed is a phospholipid of animal or vegetable origin, and more particularly one derived from hen's egg yolk or soybean.

11. Use of the fat emulsion according to claims 1 to 10, characterized in that it contains further emulsifying auxiliary materials such as sodium salts of longchain fatty acids (preferably in a concentration of from 0.005 to 0.1% by weight, based on the total emulsion) and/or cholesterol or cholesterol esters (preferably in a concentration of from 0.005 to 0.1% by weight, based on the total emulsion).

12. Use of the fat emulsion according to claims 1 to 11, characterized in that it contains $\alpha$-tocopherol or $\alpha$-tocopherol ester in an amount of from 0 to 100 mg, based on 100 g of fat.

13. Use of the fat emulsion according to claims 1 to 12, characterized in that it contains ascorbic acid or ascorbic acid ester in an amount of from 0 to 500 mg, based on 100 g of fat.

14. Use of the fat emulsion according to claims 1 to 13, characterized in that it has a pH value within the range of from 6 to 9.

## Revendications

1. Utilisation d'une émulsion (de matière) grasse contenant des acides $\omega$-3 , en particulier l'acide eicosa pentaénoïque (EPA), ou leurs esters physiologiquement inoffensifs, comme constituants de la phase grasse, cette émulsion contenant :
   - des acides gras $\omega$-3, en particulier EPA, ou leurs esters physiologiquement inoffensifs, sous forme pure ou sous forme de constituant d'huiles de poisson ou de fractions d'huiles de poisson,
   - au moins un émulsifiant physiologiquement inoffensif ,
   - éventuellement d'autres matières grasses comme des triglycérides à longueur moyenne de chaîne (TMC),
   - éventuellement l'$\alpha$-tocopherol ou des esters physiologiquement inoffensifs de l'$\alpha$-tocopherol,
   - éventuellement l'acide ascorbique ou des esters physiologiquement inoffensifs de l'acide ascorbique, ainsi que
   - des additifs et adjuvants usuels,
   - la teneur totale en matière grasse se situant entre 5 et 20 % et la teneur en émulsifiant se situant entre 5 et 12 % (par rapport à la teneur en matière grasse),
   pour préparer un médicament destiné au traitement prophylatique et thérapeutique intrapéritonéal de maladies septiques de la cavité abdominale.

2. Utilisation de l'émulsion de matière grasse selon la revendication 1 pour traiter dans la zone abdominale après des traumatismes et après des opérations.

3. Utilisation de l'émulsion de matière grasse selon la revendication 1 pour traiter une septicémie, une péritonite et une pancréatite aigue avec nécrose hémorragique.

4. Utilisation de l'émulsion de matière grasse selon la revendication 1 pour traiter dans le cadre des traitements de lavage intra-abdominal ainsi que pour les drainages de lavage.

5. Utilisation de l'émulsion de matière grasse selon la revendication 1 pour améliorer une perfusion splanchnique après des lésions opératoires et des traumatismes.

6. Utilisation de l'émulsion de matière grasse selon la revendication 1 pour le traitement prophylatique d'une défaillance rénale après des interventions chirurgicales.

7. Utilisation de l'émulsion de matière grasse selon les revendications 1 à 6, caractérisée en ce que l'huile de poisson est un concentré hautement purifié d'huile de poisson ayant une teneur en EPA qui est avantageusement au moins égale à 25 % (calculée par rapport aux esters-méthyliques d'acide gras du concentré d'huile de poisson).

8. Utilisation de l'émulsion de matière grasse selon les revendications 1 à 7, caractérisée en ce qu'on peut obtenir cette huile de poisson/ce concentré d'huile de poisson en traitant des poissons d'eau froide, par exemple des maquereaux, des sardines, des harengs ou du saumon.

9. Utilisation de l'émulsion de matière grasse selon les revendications 1 à 8, caractérisée en ce que les triglycérides à longueur moyenne de chaîne que l'on utilise éventuellement comme constituant gras consistent pour au moins 90% en glycérides de l'acide caprylique (en $C_8$) et de l'acide caprique (en $C_{10}$) et en ce que la teneur en ces constituants représente de 0 à 90 % (par rapport à la fraction lipophile).

10. Utilisation de l'émulsion de matière grasse selon les revendications 1 à 9, caractérisée en ce que l'émulsifiant utilisé est un phospholipide d'origine animale ou végétale, notamment obtenu à partir de jaune d'oeuf de poule ou de soja.

11. Utilisation de l'émulsion de matière grasse selon les revendications 1 à 10, caractérisée en ce qu'elle contient d'autres adjuvants émulsifiants comme des sels sodiques d'acides gras à longue chaîne (avantageusement 0,005 à 0,1 % en poids, par rapport à l'émulsion totale) et/ou du cholestérol ou des esters de cholestérol (de préférence 0,005 à 0,1 % en poids, par rapport à l'émulsion totale).

12. Utilisation de l'émulsion de matière grasse selon les revendications 1 à 11, caractérisée en ce qu'elle contient 0 à 100 mg d'$\alpha$-tocophérol ou d'un ester d'$\alpha$-tocophérol, pour 100 g de matière grasse.

13. Utilisation de l'émulsion de matière grasse selon les revendications 1 à 12, caractérisée en ce que l'émulsion contient 0 à 500 mg d'acide ascorbique ou d'un ester d'acide ascorbique, pour 100 g de matière grasse.

14. Utilisation de l'émulsion de matière grasse selon les revendications 1 à 13, caractérisée en ce que l'émulsion présente une valeur du pH dans la gamme de 6 à 9.